# EUROPEAN PATENT SPECIFICATION

(11) **EP 3 860 446 B1**
(45) Date of publication and mention of the grant of the patent: **17.04.2024**
(21) Application number: 19887713.6
(22) Date of filing: 12.08.2019
(51) Int. Cl.: A61B 5/021, A61B 5/024, A61B 5/00, A61B 5/11

(54) **ELECTRONIC DEVICE FOR OBTAINING BLOOD PRESSURE VALUE USING PULSE WAVE VELOCITY ALGORITHM AND METHOD FOR OBTAINING BLOOD PRESSURE VALUE**
ELEKTRONISCHE VORRICHTUNG ZUR GEWINNUNG EINES BLUTDRUCKWERTES UNTER VERWENDUNG EINES PULSWELLENGESCHWINDIGKEITSALGORITHMUS UND VERFAHREN ZUR GEWINNUNG DES BLUTDRUCKWERTES
DISPOSITIF ÉLECTRONIQUE POUR OBTENIR LA VALEUR DE LA PRESSION ARTÉRIELLE EN UTILISANT L'ALGORITHME DE LA VITESSE DE L'ONDE DE POULS ET MÉTHODE POUR OBTENIR LA VALEUR DE LA PRESSION ARTÉRIELLE

(30) Priority: 23.11.2018 KR 20180145932
(43) Date of publication of application: 11.08.2021
(73) Proprietor: Samsung Electronics Co., Ltd., Suwon-si 16677 (KR)
(72) Inventor: CHOE, Jungwoo, Suwon-si Gyeonggi-do 16677 (KR)
(74) Representative: Gulde & Partner
(86) International application number: PCT/KR2019/010215
(87) International publication number: WO 2020/105841

(56) References cited:
- WO-A1-2016/065476
- WO-A1-2018/013569
- US-A1- 2017 007 125
- US-A1- 2017 007 184
- US-A1- 2018 317 854

## Description

### [Technical Field]

The disclosure relates to a technology for obtaining a blood pressure value based on a pulse wave velocity (PWV) algorithm.

### [Background Art]

An electronic device that measures a blood pressure in a pulse wave velocity (PWV) manner may include an electrocardiography (ECG) sensor and a photoplethysmography (PPG) sensor, and may measure a blood pressure value based on signals obtained from the ECG sensor and the PPG sensor, respectively. For example, the electronic device may detect a time point when an electrical stimulation of the ventricles of the heart starts by using the ECG sensor, electrodes of which contact a plurality of portions of a user's body, and may detect a time point when a pulse wave generated in systole arrives at another portion of the user's body from the heart by using the PPG sensor facing another portion of the user's body. The electronic device may obtain a pulse wave velocity based on a time interval between the detected time points and may measure a blood pressure value by using the pulse wave velocity on a formula. Electronic devices that measure a blood pressure are disclosed e.g. in US 2017/007125 A1 and WO 2018/013569 A1

The above information is presented as background information only to assist with an understanding of the disclosure. No determination has been made, and no assertion is made, as to whether any of the above might be applicable as prior art with regard to the disclosure.

### [Disclosure]

### [Technical Problem]

Because the ECG sensor requires a plurality of electrodes, the ECG sensor occupies a much mounting space of the electronic device, and there is a risk of corrosion when the plurality of electrodes are exposed to the outside and frequently come into contact with water. In addition, it is expensive to implement the ECG sensor.

### [Technical Solution]

Aspects of the disclosure are to address at least the above-mentioned problems and/or disadvantages and to provide at least the advantages described below. Accordingly, an aspect of the disclosure is to provide an electronic device that obtains a blood pressure value by using a PWV algorithm only with a plurality of PPG sensors without an ECG sensor and a blood pressure value obtaining method of the electronic device.

Additional aspects will be set forth in part in the description which follows and, in part, will be apparent from the description, or may be learned by practice of the presented embodiments.

The invention is defined by the appended claims. The description that follows is subjected to this limitation. Any disclosure lying outside the scope of said claims is only intended for illustrative as well as comparative purposes.

Other aspects, advantages, and salient features of the disclosure will become apparent to those skilled in the art from the following detailed description, which, taken in conjunction with the annexed drawings, discloses various embodiments of the disclosure.

### [Advantageous Effects]

According to embodiments of the disclosure, a blood pressure value may be calculated based on a PWV algorithm by using only a PPG sensor without an ECG sensor. Besides, a variety of effects directly or indirectly understood through this disclosure may be provided.

### [Description of Drawings]

The above and other aspects, features, and advantages of certain embodiments of the disclosure will be more apparent from the following description taken in conjunction with the accompanying drawings, in which:
FIG. 1 illustrates a usage environment of a first electronic device and a second electronic device according to an embodiment of the disclosure;
FIG. 2 illustrates the exterior of housings of a first electronic device and a second electronic device according to an embodiment of the disclosure;
FIG. 3 is a configuration diagram of a first electronic device (master device) according to an embodiment of the disclosure;
FIG. 4 is a configuration diagram of a second electronic device (slave device) according to an embodiment of the disclosure;
FIG. 5 is a flowchart of a blood pressure value obtaining method of an electronic device according to an embodiment of the disclosure;
FIG. 6 illustrates an allowable blood pressure range setting method of an electronic device according to an embodiment of the disclosure;
FIG. 7 is an example in which an electronic device displays guide information associated with obtaining a blood pressure, according to an embodiment of the disclosure;
FIG. 8 indicates an example of a first electronic device or a second electronic device according to an embodiment of the disclosure;
FIG. 9 illustrates a graph for describing a method for obtaining a blood pressure based on an ECG sensor of a first electronic device and a PPG signal, according to an embodiment of the disclosure; and
FIG. 10 is a block diagram illustrating an electronic device in a network environment according to various embodiments of the disclosure.

Throughout the drawings, like reference numerals will be understood to refer to like parts, components, and structures.

### [Best Mode]

The following description with reference to the accompanying drawings is provided to assist in a comprehensive understanding of various embodiments of the disclosure as defined by the claims. It includes various specific details to assist in that understanding but these are to be regarded as merely exemplary. In addition, descriptions of well-known functions and constructions may be omitted for clarity and conciseness.

The terms and words used in the following description and claims are not limited to the bibliographical meanings, but, are merely used by the inventor to enable a clear and consistent understanding of the disclosure. Accordingly, it should be apparent to those skilled in the art that the following description of various embodiments of the disclosure is provided for illustration purpose only and not for the purpose of limiting the disclosure as defined by the appended claims.

It is to be understood that the singular forms "a," "an," and "the" include plural referents unless the context clearly dictates otherwise. Thus, for example, reference to "a component surface" includes reference to one or more of such surfaces.

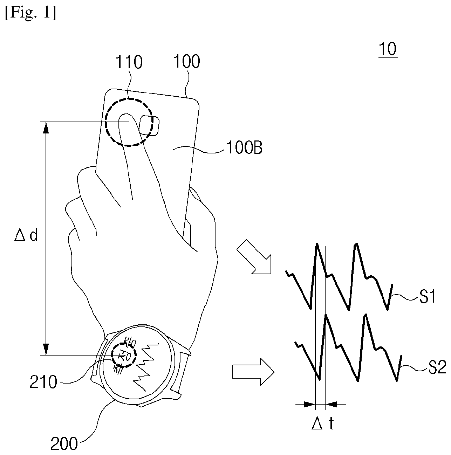

FIG. 1 illustrates a usage environment of a first electronic device and a second electronic device according to an embodiment of the disclosure.

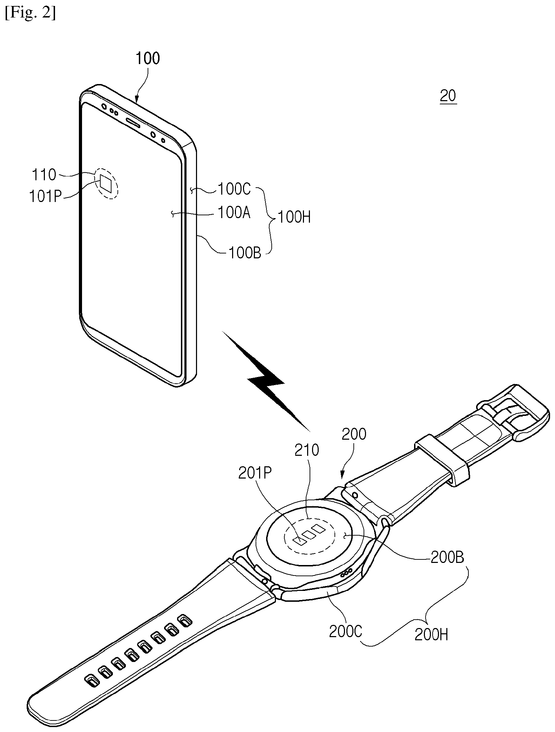

FIG. 2 illustrates the exterior of a first electronic device and a second electronic device according to an embodiment of the disclosure.

Referring to FIGS. 1 and 2, according to an embodiment, a first electronic device 100 may include a housing 100H and a first PPG sensor 110, as shown in usage environment 10. The housing 100H may include a first surface 100B (e.g., a back surface), a second surface 100A (e.g., a front surface) facing away from the first surface 100B, and a side surface 100C surrounding a space between the first surface 100B and the second surface 100A. A first portion 101P of the housing 100H may be a substantially transparent portion formed in the first surface 100B of the housing 100H. At least a portion (e.g., a light emitting unit) of the first PPG sensor 110 may be exposed through the first portion 101P of the housing 100H, and the first PPG sensor 110 may face a first body portion (e.g., an index fingertip) of a user. The first electronic device 100 may emit a light to the first body portion of the user by using the first PPG sensor 110 and may generate a first pulse signal S1 corresponding to a pulse wave of the user, which passes through the first body portion, based on a reflection light of the emitted light, as shown in usage environment 10.

According to an embodiment, the exterior 20 of the first electronic device and the second electronic device may each include a housing and a PPG sensor. According to an embodiment, a second electronic device 200 may include a housing 200H and a second PPG sensor 210. The housing 200H may include a first surface 200B (e.g., a back surface), a second surface (not illustrated) (e.g., a front surface) facing away from the first surface 200B, and a side surface 200C surrounding a space between the first surface 200B and the second surface (not illustrated). A first portion 201P of the housing 200H may be a substantially transparent portion formed in the first surface 200B of the housing 200H. At least a portion (e.g., a light emitting unit) of the second PPG sensor 210 may be exposed through the first portion 201P of the housing 200H, and the second PPG sensor 210 may face a second body portion (e.g., a wrist) of the user. The second electronic device 200 may emit a light to the second body portion of the user by using the second PPG sensor 210 and may generate a second pulse signal S2 corresponding to a pulse wave of the user, which passes through the second body portion, based on a reflection light of the emitted light, as shown in usage environment 10.

According to an embodiment, one electronic device (e.g., the first electronic device 100) of the first electronic device 100 and the second electronic device 200 may operate as a master device, and the other electronic device (e.g., the second electronic device 200) may operate as a slave device. The master device may be an electronic device, which receives an input associated with a request for calculating (or, obtaining) a blood pressure (hereinafter referred to as a "blood pressure calculating request"), from among the first electronic device 100 and the second electronic device 200; an electronic device, which includes a processor having a relatively high performance, from among the first electronic device 100 and the second electronic device 200; or an electronic device, which is equipped with an output device, from among the first electronic device 100 and the second electronic device 200. In this disclosure, for convenience of description, the case where the first electronic device 100 operates as a master device and the second electronic device 200 operates as a slave device will be described as an example.

The first electronic device 100 (master device) may generate the first pulse signal S1 by using the first PPG sensor 110 depending on an input associated with the blood pressure calculating request, may request the second pulse signal S2, which the second electronic device 200 (slave device) generates, from the second electronic device 200, and may receive the second pulse signal S2 from the second electronic device 200. The first electronic device 100 may calculate a time difference ×t between the first pulse signal S1 and the second pulse signal S2 and may calculate a pulse wave velocity (PWV) based on the calculated time difference ×t and a distance ×d between the first body portion and the second body portion. Also, the first electronic device 100 may calculate a blood pressure value of the user based on the calculated pulse wave velocity, for example, by putting the pulse wave velocity into a specified formula for calculating a blood pressure value.

According to an embodiment, the first electronic device 100 may communicate with the second electronic device 200 so as to synchronize the first pulse signal S1 and the second pulse signal S2. For example, the first electronic device 100 and the second electronic device 200 may synchronize a reference time with a time point to simultaneously receive an external light. For another example, the first electronic device 100 and the second electronic device 200 may synchronize the reference time with a time point when the first electronic device 100 and the second electronic device 200 receive the first pulse signal S1 and the second pulse signal S2 corresponding to lights reflected from two body portions of the user, which are very close to each other (e.g., within about 3 cm), after the first electronic device 100 and the second electronic device 200 simultaneously emit lights to the two body portions of the user by using the first PPG sensor 110 and the second PPG sensor 210. For another example, in the case where the first electronic device 100 and the second electronic device 200 are set to the universal standard time, the process of synchronizing the reference time may be skipped. Below, the case where the reference time between the first electronic device 100 and the second electronic device 200 is synchronized will be described as an example.

According to an embodiment, the first electronic device 100 or the second electronic device 200 may be a device such as a smartphone, a wrist band-type device, a smart watch, an earphone, smart glasses, or smart clothes.

According to various embodiments, the first electronic device 100 (master device) may receive a plurality of pulse signals from a plurality of slave devices, respectively, and may calculate a blood pressure value based on a time difference between the plurality of pulse signals. For example, the plurality of slave devices may include the second electronic device 200 and a third electronic device (not illustrated) (including a third PPG sensor facing a third body portion). In this case, the first electronic device 100 may generate a first pulse signal and may receive a second pulse signal and a third pulse signal from the second electronic device 200 and the third electronic device (not illustrated), respectively. The first electronic device 100 may calculate a blood pressure value based on a time difference between the first pulse signal and the second pulse signal, and may calculate a blood pressure value based on a time difference between the first pulse signal and the third pulse signal, and may calculate a blood pressure value based on a time difference between the second pulse signal and the third pulse signal.

According to various embodiments, the calculation of the blood pressure value based on the first pulse signal and the second pulse signal may be performed by a device (e.g., a server device) except for the first electronic device 100 and the second electronic device 200. In this case, the first electronic device 100 and the second electronic device 200 may respectively transmit the first pulse signal and the second pulse signal to the server device, and one electronic device (master device) of the first electronic device 100 and the second electronic device 200 may receive a blood pressure value calculated by the server device from the server device and may provide (e.g., display) the received blood pressure value to the user.

In the above embodiment, the case where the first electronic device 100 is already set to a master device is described as an example. However, the disclosure is not limited thereto. For example, when the first electronic device 100 receives an input associated with the blood pressure calculating request, the first electronic device 100 may communicate with the second electronic device 200 to determine whether to operate as a master device or whether to operate as a slave device and may operate as a master device or a slave device depending on the determination. In this process, the first electronic device 100 and the second electronic device 200 may determine, as a master device, an electronic device that corresponds to at least one of an electronic device directly receiving an input associated with the blood pressure calculating request, an electronic device including a processor having a relatively high performance, or an electronic device equipped with an output device (e.g., a display). The other electronic device, which is not determined as a master device, from among the first electronic device 100 and the second electronic device 200 may be determined as a slave device.

According to various embodiments, at least one of the first electronic device 100 and the second electronic device 200 may include another sensor (e.g., an ECG sensor) capable of measuring a biometric signal. In this case, a processor 170 may allow another sensor (e.g., an ECG sensor) to be disabled when measuring a first blood pressure value by at least using the first pulse signal and the second pulse signal.

According to the above embodiment, the first electronic device 100 may estimate a blood pressure value in the PWV manner by using only a PPG sensor without using an ECG sensor. As such, in the above embodiment, a space necessary to dispose components for calculating a blood pressure value and costs may be reduced, and an issue such as corrosion of an electrode that is exposed to the outside may be prevented.

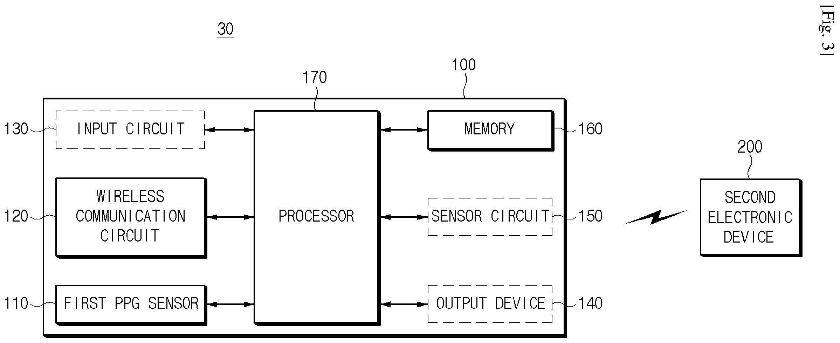

FIG. 3 is a configuration diagram of a first electronic device (master device) according to an embodiment of the disclosure.

Referring to FIG. 3, according to an embodiment, the first electronic device 100 (e.g., the first electronic device 100 of FIG. 1) may include the first PPG sensor 110 (e.g., the first PPG sensor 110 of FIG. 1), a wireless communication circuit 120, a memory 160, and the processor 170, as shown in configuration diagram 30. In an embodiment, the first electronic device 100 may not include some of the components or may further include any other additional component. For example, the first electronic device 100 may include an input circuit 130, an output device 140, and a sensor circuit 150. In an embodiment, some components of the first electronic device 100 may be combined to form one entity, which identically performs functions of the corresponding components before the combination.

According to an embodiment, the first PPG sensor 110 may face the first body portion (e.g., an index fingertip) of the user through a first portion (e.g., the first portion 101P of FIG. 2) of the first electronic device 100 (e.g., the housing 100H FIG. 2). The first PPG sensor 110 may emit a light to the first body portion of the user depending on a command of the processor 170 and may generate the first pulse signal S1 corresponding to a pulse wave of the user based on a reflection light of the emitted light. According to various embodiments, the first PPG sensor 110 may pre-process (e.g., take a second-order derivative of) the first pulse signal S1 corresponding to a change in the intensity of the reflection light. In this case, the processor 170 may receive the pre-processed first pulse signal.

According to an embodiment, the wireless communication circuit 120 may establish a wireless communication channel of a specified communication manner. The specified communication manner may be, for example, a short-range communication manner such as Wi-Fi, Bluetooth, or Zigbee.

According to an embodiment, the input circuit 130 may sense or receive a user input. The input circuit 130 may include at least one of a physical button, a touch sensor, or a microphone.

According to an embodiment, the output device 140 may include at least one of a display, a speaker, or a vibration element. The display may display, for example, various kinds of content (e.g., a text, an image, a video, an icon, and/or a symbol). The display may be a touchscreen display combined with the input circuit 130. In this disclosure, the case where the output device 140 includes a display is described as an example.

For example, the memory 160 may store a command or data associated with at least one other component of the first electronic device 100. According to an embodiment, the memory 160 may store instructions that, when executed, the processor 170 to obtain the first pulse signal corresponding to a pulse wave measured from the first body portion by using the first PPG sensor 110, to receive the second pulse signal corresponding to a pulse wave, which the second electronic device 200 measures at the second body portion by using the second PPG sensor 210, from the second electronic device 200 (an external electronic device) through the wireless communication circuit 120, to calculate a time difference between the first pulse signal and the second pulse signal, to calculate a first blood pressure value using a pulse wave velocity (PWV) algorithm based on the calculated time difference, and to provide the first blood pressure value through the output device 140.

The processor 170 may perform data processing or an operation associated with a control and/or a communication of at least one other component(s) of the first electronic device 100 by using the instructions stored in the memory 160. For example, the processor 170 may include at least one of a central processing unit (CPU), a graphics processing unit (GPU), a microprocessor, an application processor (AP), an application specific integrated circuit (ASIC), or a field programmable gate arrays (FPGA) and may include a plurality of cores.

According to an embodiment, the processor 170 may receive (or sense) an input associated with the blood pressure calculating request through the input circuit 130. Alternatively, the processor 170 may receive an input associated with the blood pressure calculating request from the second electronic device 200 through the wireless communication circuit 120. When the processor 170 receives the input associated with the blood pressure calculating request, the processor 170 may emit a light to the first body portion by using the first PPG sensor 110 and may generate the first pulse signal based on a reflection light. The processor 170 may communicate with the second electronic device 200 through the wireless communication circuit 120 to transmit a request associated with generating the second pulse signal to the second electronic device 200, and may receive the second pulse signal from the second electronic device 200 as a response to the request.

According to an embodiment, the processor 170 may calculate a time difference between the generated first pulse signal and the received second pulse signal and may calculate the first blood pressure value by using the PWV algorithm based at least on the calculated time difference. The first blood pressure value may include, for example, at least one of a diastolic blood pressure value, a systolic blood pressure value, or an average blood pressure value.

According to an embodiment, the processor 170 may obtain a distance between the first electronic device 100 and the second electronic device 200 from the memory 160, may calculate a pulse wave velocity by using the obtained distance and the calculated time difference, and may calculate the first blood pressure value on a formula by using the calculated pulse wave velocity. The distance may be determined based on a user input and may be stored in the memory 160. The user input may include a distance between the first body portion and the second body portion or information of the first body portion and the second body portion. When the information of the first body portion and the second body portion is input, the processor 170 may determine a distance based on body information (e.g., a height and a weight) of the user stored in the memory 160 and may store the distance in the memory 160.

According to an embodiment, the processor 170 may calculate the first blood pressure value corresponding to the calculated time difference based on relationship information between a reference blood pressure value and a reference time difference. The reference blood pressure value may be, for example, a blood pressure value (mmHg) (e.g., a diastolic blood pressure value and a systolic blood pressure value) that is measured by a sphygmomanometer (or a blood pressure meter) and is set (or input) to the first electronic device 100. For another example, the reference blood pressure value may be determined and set by the processor 170, based on personal information (e.g., an age, a sex, a race, a height, a weight, and a pulse rate) of the user. The first blood pressure value may include, for example, at least one of a diastolic blood pressure value, a systolic blood pressure value, or an average blood pressure value. After the reference blood pressure value is set or while the reference blood pressure value is being measured, the processor 170 may generate the first pulse signal by using the first PPG sensor 110 and may receive the second pulse signal from the second electronic device 200. The processor 170 may verify a reference time difference between the first pulse signal and the second pulse signal by monitoring the first pulse signal and the second pulse signal. The processor 170 may verify a formula relationship between a time difference and a blood pressure value based on the reference time difference and the reference blood pressure value and may store information about the verified relationship between the time difference and the blood pressure value in the memory 160 (a calibration process). Afterwards, the processor 170 may monitor the first pulse signal and the second pulse signal by using the first PPG sensor 110 and the second PPG sensor 210, may verify a time difference between the first pulse signal and the second pulse signal thus obtained, and may calculate the first blood pressure value corresponding to the verified time difference based on the information about the verified relationship between the time difference and the blood pressure value (an actual blood pressure measuring process).

According to an embodiment, when the first blood pressure value is calculated, the processor 170 may display guide information associated with the first blood pressure value calculated, through the output device 140 (an user interface). For example, the processor 170 may display at least one of a number or an icon indicating the first blood pressure value through the output device 140. For another example, the first electronic device 100 may calculate the first blood pressure value including at least one of a diastolic blood pressure value, a systolic blood pressure value, or an average blood pressure value based on the calculated time difference and may display guide information associated with the first blood pressure value.

According to an embodiment, the processor 170 may compare the first blood pressure value and the reference blood pressure to determine whether a blood pressure is abnormal. For example, when the first blood pressure value is within an allowable blood pressure range, the processor 170 may determine that the blood pressure of the user is in a normal state. Also, when the calculated first blood pressure value is out of the allowable blood pressure range, the processor 170 may determine that the blood pressure of the user is in an abnormal state. The processor 170 may display guide information about a normal state or an abnormal state of a blood pressure through the output device 140.

In an embodiment, the processor 170 may differently set the allowable blood pressure range based on at least one of personal information (e.g., a sex, an age, a race, a height, and a weight) of the user, ambient environment information (e.g., a noise, a temperature, a humidity, and an altitude), or health-related information (e.g., an exercise habit, a sleep habit, a dietary habit, stress status, and a medicine that the user is taking). For example, the processor 170 may set the allowable blood pressure range based on a statistical blood pressure value according to an age, a sex, a race, or a body mass index (determined based on a height and a weight) that is input through the input circuit 130 and is stored in the memory 160. For another example, when a lack of exercise, a lack of sleep, an irregular dietary situation, or a stress situation is verified based on the health-related information that is input through the input circuit 130 or is verified through the sensor circuit 150, the processor 170 may set the allowable blood pressure range to be relatively high. Alternatively, when a situation requiring attention to blood pressure care is verified based on the health-related information (in the case of taking a blood pressure medicine), the processor 170 may set the allowable blood pressure range to be lower. For another example, when the altitude verified based on information about an ambient environment sensed through the sensor circuit 150 is a specified altitude or higher, when the verified noise is a specified level or higher, when the verified humidity is a specified humidity or higher, or when the verified temperature is lower than a specified temperature, the processor 170 may set the allowable blood pressure range to be high.

According to an embodiment, the processor 170 may calculate any other blood pressure values, for example, a second blood pressure value and a third blood pressure value by using the first pulse signal and the second pulse signal, respectively. For example, the processor 170 may calculate the second blood pressure value by analyzing the first pulse signal by using a pulse wave analysis (PWA) algorithm. The processor 170 may calculate the third blood pressure value by analyzing the second pulse signal by using the PWA algorithm. According to various embodiments, the first electronic device 100 may further include an ECG sensor, and may calculate the second blood pressure value by using the first pulse signal and any other signal obtained by using the ECG sensor.

The processor 170 may verify at least one of the accuracy of blood pressure calculation or cardiovascular health status based on at least one of the second blood pressure value or the third blood pressure value. For example, when a difference between the first blood pressure value and the second blood pressure value is within a first error, the processor 170 may determine that the first blood pressure value is accurately calculated. Additionally or alternatively, when a difference between the first blood pressure value and the third blood pressure value is within a second error, the processor 170 may determine that the first blood pressure value is accurately calculated. The first and second errors may be determined, for example, based on an error between a blood pressure value calculated by using the PWV algorithm and a blood pressure value calculated by using the PWA algorithm. For another example, when a difference between the second blood pressure value and the third blood pressure value is a third error or greater, the processor 170 may determine that the cardiovascular status of the user is abnormal. The third error may be determined based on a specified index indicating abnormal cardiovascular status.

According to an embodiment, the processor 170 may obtain movement information of the first electronic device 100 by using the sensor circuit 150 and may determine whether it is possible to calculate a blood pressure, based on the movement information. The processor 170 may periodically obtain the movement information of the first electronic device 100 by using the sensor circuit 150 and may determine whether the movement of the first electronic device 100 is a specified strength or greater, based on the movement information. The specified strength may be, for example, a reference for determining whether the user does strenuous exercise and may be determined experimentally. Afterwards, when receiving an input associated with calculating a blood pressure, the processor 170 may determine whether the input associated with calculating a blood pressure is received after there is verified that the movement of the first electronic device 100 is the specified strength or greater. When that the movement of the first electronic device 100 is the specified strength or greater is verified and the input associated with calculating a blood pressure is received, the processor 170 may guide that the calculation of a blood pressure is impossible, through the output device 140. According to an embodiment, the processor 170 may receive movement information of the second electronic device 200 and may determine whether it is possible to calculate a blood pressure, based on the received movement information. For example, when an input associated with calculating a blood pressure is received, the processor 170 may request movement information of the second electronic device 200 from the second electronic device 200, may receive movement information of the second electronic device 200 within a first specified time before the input is received from the second electronic device 200, and may determine whether it is possible to calculate a blood pressure based on the received movement information.

According to an embodiment, the processor 170 may verify a recovery speed of a blood pressure value after strenuous exercise, based on the movement information received through the sensor circuit 150. For example, the processor 170 may verify that the movement of the first electronic device 100 is a specified strength or greater based on the received movement information and may output guide information (e.g., an alarm sound and words of guidance) associated with verifying the recovery speed after exercise at a time point when a second specified time elapses. The guide information associated with verifying the recovery speed after exercise may include an alarm sound and words of guidance guiding a time to measure a blood pressure value for the purpose of verifying the recovery speed after exercise. In the case where the user checks the guide information associated with verifying the recovery speed after exercise, the user may generate the blood pressure calculating request through the input circuit 130. When the processor 170 receives an input associated with the blood pressure calculating request, the processor 170 may obtain the first pulse signal and the second pulse signal by using the first PPG sensor 110 and the second PPG sensor 210 of the second electronic device 200 and may calculate a blood pressure value based on a time difference between the first pulse signal and the second pulse signal. The processor 170 may verify a recovery speed of a blood pressure value after exercise based on the calculated blood pressure value and may display guide information associated with the verified recovery speed through the output device 140.

According to an embodiment, the processor 170 may adjust a sensitivity of the first PPG sensor 110 based on a user input. For example, when an input associated with setting the sensitivity is received through the input circuit 130, the processor 170 may emit a light by using the first PPG sensor 110 and may determine whether the intensity of a reflection light of the emitted light is within a specified sensitivity range. When the intensity of the reflection light is out of the specified sensitivity range, the processor 170 may adjust (e.g., increase or decrease) the intensity of a light to be emitted by the first PPG sensor 110 such that the light is within the specified sensitivity range. For another example, the processor 170 may adjust the intensity of a light to be emitted by the first PPG sensor 110 based on personal information (e.g., an age, a sex, and a race) of the user stored in the memory 160. For example, in the case where there is a cause to decrease the intensity of the reflection light, such as a dark skin color, a lot of hairs, or a lot of wrinkles, the processor 170 may increase the intensity of a light that the first PPG sensor 110 emits.

According to various embodiments, the processor 170 may receive, from the second electronic device 200, the third blood pressure value that the second electronic device 200 calculates based on the second pulse signal.

According to various embodiments, when an input associated with the blood pressure calculating request is received through the input circuit 130, the processor 170 may output guide information associated with calculating a blood pressure through the output device 140 in response to the input associated with the blood pressure calculating request, before generating the first pulse signal and the second pulse signal. The guide information associated with calculating a blood pressure may include, for example, information guiding a posture appropriate to calculate a blood pressure. For example, the guide information associated with calculating a blood pressure may include words or a sound that guides heights of the first body portion and the second body portion so as to be maintained to be similar to the heart.

According to various embodiments, in the case where the first electronic device 100 does not include the output device 140, the processor 170 may communicate with the second electronic device 200 including the output device 240 through the wireless communication circuit 120 and may output guide information associated with a blood pressure value through the output device 240 included in the second electronic device 200.

According to various embodiments, the processor 170 (a processor of a master device) may receive a plurality of pulse signals from a plurality of slave devices, respectively, and may calculate a blood pressure value based on a time difference between the plurality of pulse signals. For example, the plurality of slave devices may include the second electronic device 200 and a third electronic device (not illustrated) (including a third PPG sensor facing the third body portion). In this case, the processor 170 may generate the first pulse signal and may receive the second pulse signal and the third pulse signal from the second electronic device 200 and the third electronic device (not illustrated), respectively. The first electronic device 100 may calculate a blood pressure value based on a time difference between the first pulse signal and the second pulse signal, may calculate a blood pressure value based on a time difference between the first pulse signal and the third pulse signal, and may calculate a blood pressure value based on a time difference between the second pulse signal and the third pulse signal. In this regard, for example, in the calibration process, the processor 170 may store relationship information between a blood pressure value and a time difference between the first pulse signal and the third pulse signal and relationship information between a blood pressure value and a time difference between the second pulse signal and the third pulse signal in the memory 160 and may calculate a blood pressure value. Alternatively, the processor 170 may store distance information between the first body portion and the third body portion and distance information between the second body portion and the third body portion in the memory 160 and may calculate a blood pressure value by using corresponding distance information.

According to the above embodiment, the first electronic device 100 may calculate a blood pressure value in the PWV manner by using only a PPG sensor without using an ECG sensor. As such, in the above embodiment, a space necessary to dispose components for calculating a blood pressure value may be reduced, and an issue such as corrosion of an electrode that is exposed to the outside may be prevented.

According to an embodiment, an electronic device (e.g., the first electronic device 100 of FIG. 3) may include a housing (e.g., the housing 100H of FIG. 2), a first photoplethysmography (PPG) sensor (e.g., the first PPG sensor 110 of FIG. 3) that is exposed to a first portion of the housing and faces a first body portion of a user to measure a pulse wave, a wireless communication circuit (e.g., the wireless communication circuit 120) that is positioned within the housing, a processor (e.g., the processor 170 of FIG. 3) that is positioned within the housing and is operatively connected with the first PPG sensor and the wireless communication circuit, and a memory (e.g., the memory 160 of FIG. 3) that is positioned within the housing and is operatively connected with the processor. The memory may store instructions that, when executed, cause the processor to monitor a first pulse signal measured by the first PPG sensor, to receive a second pulse signal measured by an external electronic device by using the wireless communication circuit, to calculate a time difference between the first pulse signal and the second pulse signal, based at least partially on the monitored first pulse signal and the received second pulse signal, and to provide a first blood pressure value by using a pulse wave velocity (PWV) algorithm, based at least partially on the calculated time difference.

The second pulse signal may be a signal which the external electronic device measures from a second body portion spaced from the first body portion as much as a given distance by using a second PPG sensor included in the external electronic device.

The electronic device may further include a user interface (e.g., the output device 140 of FIG. 3) that is disposed at a second portion of the housing, and the instructions may cause the processor to display guide information associated with the provided first blood pressure value on the user interface.

The electronic device further includes an input circuit (e.g., the input circuit 130) that receives an input associated with a request for providing the first blood pressure value, and a sensor circuit (e.g., the sensor circuit 150 of FIG. 3) that senses a movement of the electronic device, and the instructions causes the processor to obtain movement information of the electronic device by using the sensor circuit, to determine whether the movement of the electronic device is a specified strength or greater, based at least partially on the movement information, and to guide that it is impossible to provide the first blood pressure value, through the user interface, when it is determined that the movement of the electronic device is the specified strength or greater and the input is received within a specified time.

The instructions may cause the processor to emit a light to the first body portion by using the first PPG sensor and to adjust a sensitivity of the first PPG sensor based on a reflection light of the emitted light.

The memory may store the given distance between the first body portion and the second body portion, and the instructions may cause the processor to verify the stored distance from the memory and to calculate the first blood pressure value based at least partially on the verified distance and the time difference.

According to an embodiment, an electronic device (e.g., the electronic device 100 of FIG. 3) may include an output device (e.g., the output device 140 of FIG. 3), a first PPG sensor (e.g., the first PPG sensor 110 of FIG. 3) that faces a first body portion of a user, a wireless communication circuit (e.g., the wireless communication circuit 120 of FIG. 3) that communicates with an external electronic device (e.g., the second electronic device 200 of FIG. 1), the external electronic device including a second PPG sensor (e.g., the second PPG sensor 210 of FIG. 1) that faces a second body portion of the user, which is spaced from the first body portion as much as a given distance, a processor (e.g., the processor 170 of FIG. 3) that is operatively connected with the first PPG sensor and the wireless communication circuit, and a memory (e.g., the memory 160 of FIG. 3) that is operatively connected with the processor. The memory may store instructions that, when executed, cause the processor to obtain a first pulse signal corresponding to a pulse wave measured at the first body portion by using the first PPG sensor, to receive a second pulse signal corresponding to a pulse wave, which the external electronic device measures at the second body portion by using the second PPG sensor, from the external electronic device through the wireless communication circuit, to calculate a time difference between the obtained first pulse signal and the received second pulse signal, to calculate a first blood pressure value by using a pulse wave velocity (PWV) algorithm, based at least partially on the calculated time difference, and to provide the first blood pressure value through the output device.

The memory may store the given distance between the first body portion and the second body portion, and the instructions may cause the processor to verify the stored distance from the memory and to calculate the first blood pressure value based at least partially on the verified distance and the time difference.

The instructions may cause the processor to calculate the first blood pressure value corresponding to the time difference, based at least partially on relationship information between a blood pressure value and the time difference.

The instructions may cause the processor to estimate a second blood pressure value by using a PWA algorithm based on at least one pulse signal of the first pulse signal or the second pulse signal and to verify an accuracy of the first blood pressure value based at least partially on the second blood pressure value.

The instructions may cause the processor to estimate a second blood pressure value by using a pulse wave analysis (PWA) algorithm, based on the first pulse signal, to obtain a third blood pressure value estimated by using the PWA algorithm based on the second pulse signal received from the external electronic device, and to verify at least one of an accuracy of the first blood pressure value or an abnormal cardiovascular status of the user based at least partially on the second blood pressure value or the third blood pressure value.

The instructions may cause the processor to emit a light to the first body portion by using the first PPG sensor and to adjust a sensitivity of the first PPG sensor based on a reflection light of the emitted light.

The electronic device further includes an input circuit (e.g., the input circuit 130 of FIG. 3) that receives an input of the user, and a sensor circuit (e.g., the sensor circuit 150 of FIG. 3) that senses a movement of the electronic device, and the instructions causes the processor to obtain movement information of the electronic device by using the sensor circuit, to determine whether the movement of the electronic device is a specified strength or greater, based at least partially on the obtained movement information, to receive an input associated with a blood pressure calculating request by using the input circuit, and to guide that it is impossible to calculate a blood pressure, through the output device, when the input is received within a specified time after it is determined that the movement of the electronic device is the specified strength or greater.

The instructions may cause the processor to communicate with the external electronic device by using the wireless communication circuit to receive the second pulse signal synchronized with the first pulse signal.

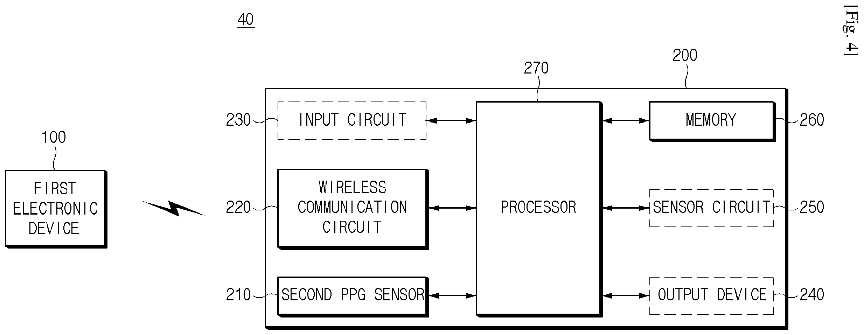

FIG. 4 is a configuration diagram of a second electronic device (slave device) according to an embodiment of the disclosure.

Referring to FIG. 4, the second electronic device 200 (e.g., the second electronic device 200 of FIG. 1) may include a wireless communication circuit 220, the second PPG sensor 210 (e.g., the second PPG sensor 210 of FIG. 1), a memory 260, and a processor 270, as shown in configuration diagram 40. In an embodiment, the second electronic device 200 may not include some of the components or may further include any other additional component. For example, the second electronic device 200 may include an input circuit 230, an output device 240, and a sensor circuit 250. In an embodiment, some components of the second electronic device 200 may be combined to form one entity, which identically performs functions of the corresponding components before the combination.

According to an embodiment, the second PPG sensor 210 may face the second body portion (e.g., a wrist) of the user through a first portion (e.g., the first portion 201P of FIG. 2) of the second electronic device 200 (e.g., the housing 200H of FIG. 2). The second PPG sensor 210 may emit a light to the second body portion of the user depending on a command of the processor 270 and may generate the second pulse signal S2 corresponding to a pulse wave of the user based on a reflection light of the emitted light. The second PPG sensor 210 may pre-process (e.g., take a second order derivative of) the second pulse signal S2 corresponding to a change in the intensity of the reflection light. In this case, the processor 270 may receive the pre-processed second pulse signal.

According to an embodiment, the wireless communication circuit 220 may establish a wireless communication channel of a specified communication manner. The specified communication manner may be, for example, a short-range communication manner such as Wi-Fi, Bluetooth, or Zigbee.

According to an embodiment, the input circuit 230 may sense or receive a user input. The input circuit 230 may include at least one of a physical button, a touch sensor, or a microphone.

According to an embodiment, the output device 240 may include at least one of a display, a speaker, or a vibration element. The display may display, for example, various kinds of content (e.g., a text, an image, a video, an icon, and/or a symbol). The display may be a touchscreen display combined with the input circuit 230.

For example, the memory 260 may store a command or data associated with at least one other component of the second electronic device 200. According to an embodiment, the memory 260 may store instructions for obtaining the second pulse signal corresponding to a pulse wave measured from the second body portion by using the second PPG sensor 210 and transmitting the second pulse signal to the first electronic device 100 through the wireless communication circuit 220, when a request associated with generating the second pulse signal is received from the first electronic device 100.

The processor 270 may perform data processing or an operation associated with a control and/or a communication of at least one other component(s) of the second electronic device 200 by using the instructions stored in the memory 260. For example, the processor 270 may include at least one of a central processing unit (CPU), a graphics processing unit (GPU), a microprocessor, an application processor (AP), an application specific integrated circuit (ASIC), or a field programmable gate arrays (FPGA) and may include a plurality of cores.

According to an embodiment, when receiving an input associated with the blood pressure calculating request through the input circuit 230, the processor 270 may transmit the input associated with the blood pressure calculating request to the first electronic device 100 through the wireless communication circuit 220. In this case, the first electronic device 100 may receive the input associated with the blood pressure calculating request and may transmit a request associated with obtaining movement information to the second electronic device 200 for the purpose of determining whether it is possible to calculate a blood pressure.

According to an embodiment, the processor 270 may generate movement information of the second electronic device 200 by using the sensor circuit 250 and may transmit the movement information to the first electronic device 100. For example, when receiving the request associated with obtaining movement information, the processor 270 may transmit, to the first electronic device 100, movement information of a first specified time period before a time point when the request is received. In this case, the first electronic device 100 may determine whether it is possible to calculate a blood pressure, based on the movement information; when it is possible to calculate a blood pressure, the first electronic device 100 may transmit a request associated with generating the second pulse signal. For another example, the processor 270 may periodically generate the movement information of the second electronic device 200 by using the sensor circuit 250, may verify that the movement of the second electronic device 200 is a specified strength or greater, based on the movement information of the second electronic device 200, and may transmit, to the first electronic device 100, the movement information of the second electronic device 200 or situation information indicating that the movement of the second electronic device 200 is the specified strength or greater.

According to an embodiment, the processor 270 may receive the request associated with obtaining the second pulse signal from the first electronic device 100 through the wireless communication circuit 220 and may generate the second pulse signal corresponding to a pulse wave measured at the second body portion by using the second PPG sensor 210. The processor 270 may generate the second pulse signal by emitting a light during a third specified time through the second PPG sensor 210 based on the request associated with generating the second pulse signal.

According to an embodiment, the processor 270 may transmit the obtained second pulse signal to the first electronic device 100 through the wireless communication circuit 220. The processor 270 may pre-process the second pulse signal and may transmit the pre-processed second pulse signal to the first electronic device 100. The pre-processing may include, for example, at least one of noise cancellation, compression, up-sampling, or down-sampling. According to various embodiments, the processor 270 may transmit the second pulse signal to the first electronic device 100 without the pre-processing.

According to an embodiment, the processor 270 may calculate the third blood pressure value by analyzing the second pulse signal by using a pulse wave analysis (PWA) algorithm. The processor 270 may transmit the calculated third blood pressure value to the first electronic device 100 through the wireless communication circuit 220.

According to various embodiments, in the case where the second electronic device 200 includes an ECG sensor, the processor 270 may generate an ECG signal indicating a time point when an electrical stimulation of the ventricles of the heart starts by using the ECG sensor, may calculate the third blood pressure value based on the ECG signal and the second pulse signal, and may transmit the third blood pressure value to the first electronic device 100 through the wireless communication circuit 220. Alternatively, the second electronic device 200 may transmit the ECG signal and the second pulse signal to the first electronic device 100 without directly calculating the third blood pressure value. In this case, the first electronic device 100 may calculate the third blood pressure value based on the ECG signal and the second pulse signal.

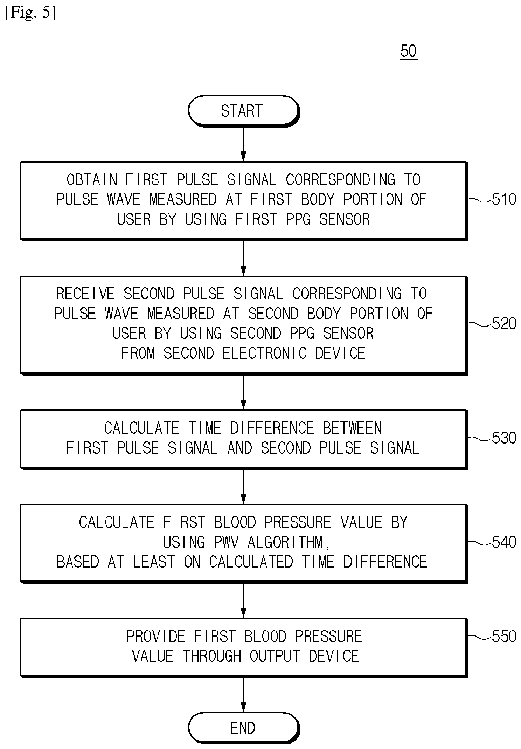

FIG. 5 is a flowchart of a blood pressure value calculating method of an electronic device according to an embodiment of the disclosure.

Referring to FIG. 5, in operation 510, the first electronic device 100 (e.g., the first electronic device 100 of FIG. 1) may obtain the first pulse signal corresponding to a pulse wave measured at the first body portion of the user by using the first PPG sensor 110 (e.g., the first PPG sensor 110 of FIG. 1), as shown in the flowchart 50. For example, the first electronic device 100 may emit a light to the first body portion by using the first PPG sensor 110, may receive a light (reflection light) reflected by the first body portion, and may obtain the first pulse signal corresponding to the reflection light. Because the intensity of the reflection light varies depending on a blood flow change by a heartbeat (associated with a blood pressure), the first electronic device 100 may obtain the first pulse signal corresponding to a pulse wave based on the reflection light.

In operation 520, the second electronic device 200 (e.g., the second electronic device 200 of FIG. 1) may obtain the second pulse signal corresponding to a pulse wave measured at the second body portion of the user by using the second PPG sensor 210. For example, the first electronic device 100 may transmit a request associated with generating the second pulse signal to the second electronic device 200 and may receive the second pulse signal from the second electronic device 200 as a response to the request.

In operation 530, the first electronic device 100 may calculate a time difference between the first pulse signal and the second pulse signal. For example, the first electronic device 100 may detect a peak of the first pulse signal and a peak of the second pulse signal and may calculate a time difference between the detected peaks.

In operation 540, the first electronic device 100 may calculate the first blood pressure value by using the PWV algorithm, based at least on the calculated time difference. For example, the first electronic device 100 may obtain a distance between the first electronic device 100 and the second electronic device 200 from the memory 160, may calculate a pulse wave velocity by using the obtained distance and the calculated time difference, and may calculate the first blood pressure value on a formula by using the calculated pulse wave velocity. For another example, the first electronic device 100 may calculate the first blood pressure value corresponding to the calculated time difference based on relationship information between a reference blood pressure value and a reference time difference stored in the memory 160. The reference blood pressure value may be, for example, a blood pressure value (mmHg) (e.g., a diastolic blood pressure value and a systolic blood pressure value) that is measured by a sphygmomanometer (or a blood pressure meter) and is set (or input) to the first electronic device 100. For another example, the reference blood pressure value may be determined and set by the first electronic device 100, based on personal information (e.g., an age, a sex, a race, a height, a weight, and a pulse rate) of the user.

In operation 550, the first electronic device 100 may provide the calculated first blood pressure value through the output device 140. For example, the first electronic device 100 may display at least one of a number or an icon indicating the first blood pressure value through the output device 140.

According to an embodiment, a method of calculating a blood pressure by an electronic device (e.g., the first electronic device 100 of FIG. 1) may include obtaining a first pulse signal corresponding to a pulse wave measured at a first body portion of a user by using a first PPG sensor (e.g., the first PPG sensor 110 of FIG. 1) facing the first body portion, receiving, from an external electronic device including a second PPG sensor (e.g., the second PPG sensor 210 of FIG. 1), a second pulse signal corresponding to a pulse wave, which the external electronic device measures at a second body portion of the user spaced from the first body portion as much as a given distance by using the second PPG sensor, calculating a time difference between the obtained first pulse signal and the received second pulse signal, calculating a first blood pressure value, based at least on the calculated time difference, and providing the first blood pressure value through an output device.

The calculating may include verifying the given distance between the first body portion and the second body portion, and calculating the first blood pressure value based on the verified distance and the time difference.

The calculating may include calculating the first blood pressure value corresponding to the time difference, based at least partially on relationship information between a blood pressure value and the time difference.

The method may further include estimating a second blood pressure value by using a pulse wave analysis (PWA) algorithm, based on the first pulse signal, obtaining a third blood pressure value calculated by using the PWA algorithm based on the second pulse signal from the external electronic device, and verifying at least one of an accuracy of the first blood pressure value or an abnormal cardiovascular status of the user based at least partially on the second blood pressure value or the third blood pressure value.

The method further includes obtaining movement information of the electronic device by using a sensor circuit (e.g., the sensor circuit 150 of FIG. 3), determining whether a movement of the electronic device is a specified strength or greater, based at least partially on the obtained movement information, receiving an input associated with a blood pressure calculating request by using an input circuit (e.g., the input circuit 130 of FIG. 3), and guiding that it is impossible to calculate a blood pressure, through the output device, when the input is received within a specified time after it is determined that the movement of the electronic device is the specified strength or greater.

The receiving may include communicating with the external electronic device to receive the second pulse signal synchronized with the first pulse signal.

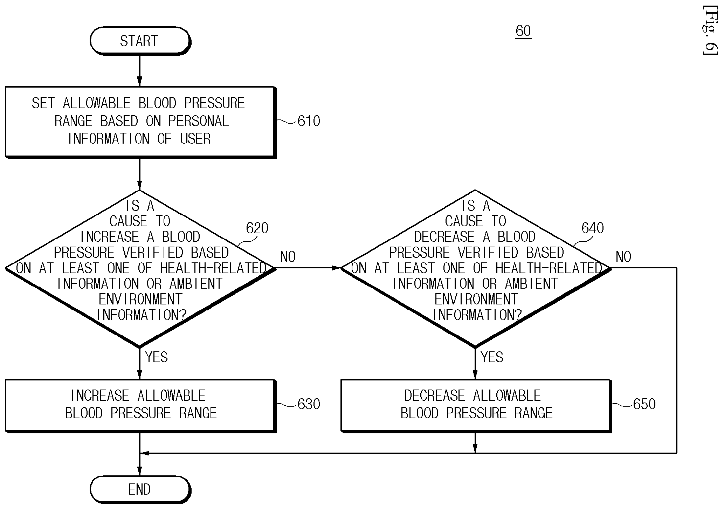

FIG. 6 illustrates an allowable blood pressure range setting method of an electronic device according to an embodiment of the disclosure.

Referring to FIG. 6, in operation 610, the first electronic device 100 (e.g., the first electronic device 100 of FIG. 1) may set the allowable blood pressure range based on personal information of the user stored in the memory 160, as shown in the flowchart 60. For example, when an input associated with the blood pressure calculating request is received, the first electronic device 100 may set the allowable blood pressure range based on the personal information of the user at least once. For another example, the first electronic device 100 may set the allowable blood pressure range based on a statistical blood pressure value according to an age, a sex, a race, or a body mass index (determined based on a height and a weight) that is input through the input circuit 130.

In operation 620, the first electronic device 100 may determine whether there is a cause to increase a blood pressure, based on at least one of health-related information or ambient environment information. For example, when a lack of exercise, a lack of sleep, or a stress situation is verified based on information (e.g., a pulse rate and movement information) sensed through at least one of the first PPG sensor 110 or the sensor circuit 150, the first electronic device 100 may verify that there is a cause to increase a blood pressure. For another example, when that an altitude is a specified altitude or higher, that a noise is a specified level or higher, that a humidity is a first specified humidity or higher, or that a temperature is a first specified temperature or higher is verified based on information received through the wireless communication circuit 120 or information (e.g., a temperature, a humidity, a noise, and an altitude) sensed through the sensor circuit 150, the first electronic device 100 may verify that there is a cause to increase a blood pressure.

When a cause to increase a blood pressure is verified based on at least one of the health-related information or the ambient environment information, in operation 630, the first electronic device 100 may increase the allowable blood pressure range based on the cause to increase a blood pressure. For example, as the number of causes to increase a blood pressure increases, the first electronic device 100 may increase the allowable blood pressure range. Afterwards, when there is verified that a cause(s) to increase a blood pressure is removed, the first electronic device 100 may again decrease the allowable blood pressure range based on the removal of the cause(s) to increase a blood pressure.

When a cause to increase a blood pressure is not verified in operation 620 based on at least one of the health-related information or the ambient environment information, in operation 640, the first electronic device 100 may determine whether there is a cause to decrease a blood pressure, based on at least one of the health-related information or the ambient environment information. For example, when that a humidity is lower than a second specified humidity or that a temperature is a second specified temperature or higher is verified based on the information received through the wireless communication circuit 120 or the information (e.g., a temperature, a humidity, a noise, and an altitude) sensed through the sensor circuit 150, the first electronic device 100 may verify that there is a cause to decrease a blood pressure. For another example, after decreasing the allowable blood pressure range due to the cause to decrease a blood pressure, the first electronic device 100 may determine whether a cause to decrease a blood pressure is removed. For another example, the first electronic device 100 may determine whether there is a situation requiring attention to blood pressure care (e.g., whether the user is taking a blood pressure medicine), based on the health-related information stored in the memory 160.

When a cause to decrease a blood pressure is verified based on at least one of the health-related information or the ambient environment information, in operation 650, the first electronic device 100 may decrease the allowable blood pressure range. For example, as the number of causes to decrease a blood pressure increases, the first electronic device 100 may decrease the allowable blood pressure range.

According to the above embodiment, the first electronic device 100 may determine the allowable blood pressure range for verifying an abnormal blood pressure based on personal information of the user, health-related information, or ambient environment information. As such, the first electronic device 100 may increase the accuracy of determination for an abnormal blood pressure.

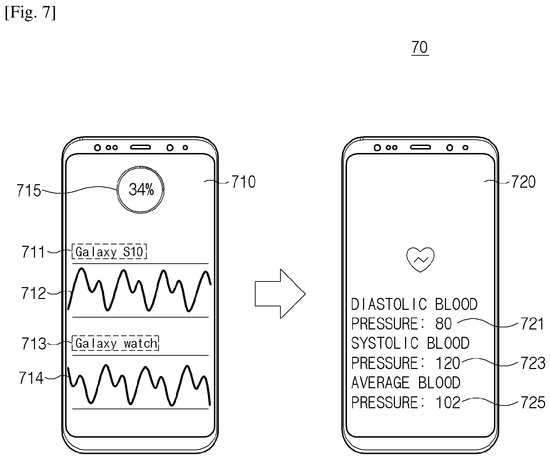

FIG. 7 is an example in which an electronic device displays guide information associated with calculating a blood pressure, according to an embodiment of the disclosure.

Referring to FIG. 7, in screen 710, the first electronic device 100 (e.g., the first electronic device 100 of FIG. 1) may display guide information associated with a process of calculating the first blood pressure value through the output device 140 (e.g., the output device 140 of FIG. 3) while calculating the first blood pressure value, as shown in the example 70. For example, the first electronic device 100 may display a graph 712 corresponding to the first pulse signal with regard to first electronic device information 711 (e.g., Galaxy S10 as a model name) and may display a graph 714 corresponding to the second pulse signal with regard to second electronic device information 713 (e.g., Galaxy Watch as a model name). For another example, the first electronic device 100 may display guide information 715 associated with a progress (%) of the calculation of the first blood pressure value.

In screen 720, when the first blood pressure value is completely calculated, the first electronic device 100 may display guide information associated with the first blood pressure value through the output device 140. For example, the guide information associated with the first blood pressure value may include at least one of a diastolic blood pressure value 721, a systolic blood pressure value 723, and an average blood pressure value 725 calculated based on a time difference.

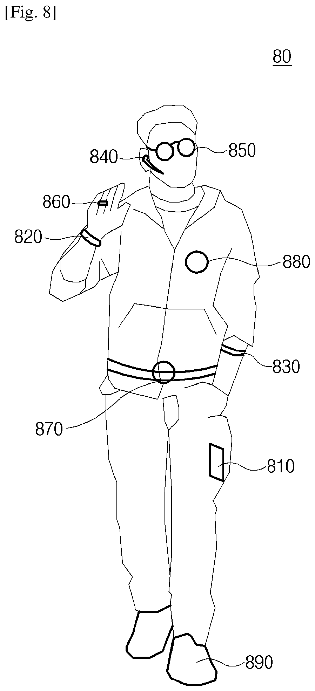

FIG. 8 indicates an example of a first electronic device or a second electronic device according to an embodiment of the disclosure.

According to an embodiment, the first electronic device 100 (e.g., the first electronic device 100 of FIG. 1) may include one of a smartphone 810, a smart watch 820, a wrist band-type device 830, an earphone 840, smart glasses 850, a smart ring or smart gloves 860, a smart belt 870, smart clothes (e.g., an electronic device included in clothes in the form of a chest patch 880), or smart shoes 890, as shown in the example 80. The smartphone 810 and the smart ring or smart gloves 860 may measure a pulse signal at a finger portion, the earphone 840 may measure a pulse signal at an ear portion, the smart glasses 850 may measure a pulse signal at a temple portion, and the smart watch 820 or the wrist band-type device 830 may measure a pulse signal at a wrist portion.

Likewise, the second electronic device 200 (e.g., the second electronic device 200 of FIG. 2) may include one of the smartphone 810, the smart watch 820, the wrist band-type device 830, the earphone 840, the smart glasses 850, the smart ring or smart gloves 860, the smart belt 870, the smart clothes (e.g., an electronic device included in clothes in the form of a chest patch 880), or the smart shoes 890.

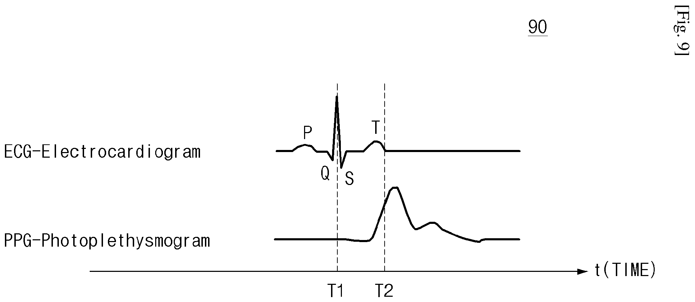

FIG. 9 illustrates a graph for describing a method for calculating a blood pressure based on an ECG sensor and a PPG signal, according to an embodiment of the disclosure.

Referring to FIG. 9, the first electronic device 100 (e.g., the first electronic device 100 of FIG. 1) may detect a time point T1 (R-peak) when an electrical stimulation of the ventricles of the heart starts, based on a pulse signal measured by using the ECG sensor, as shown in the graph 90. The first electronic device 100 may detect a time point T2 when a pulse wave arrives at the first body portion, based on a pulse signal obtained by using the first PPG sensor 110. The first electronic device 100 may calculate a pulse arrival time (PAT) being a time difference between the time point T1 when an electrical stimulation of the ventricles of the heart starts and the time point T2 when a pulse wave arrives at the first body portion.

In addition to a pulse transit time (PTT) taken for a pulse wave to arrive at the first body portion from the heart, the PAT may further include a pre-ejection period (PEP) being a time period from a start of an electrical stimulation of the ventricles of the heart to an actual open of the aortic valve. Accordingly, a blood pressure value that is calculated based on the PAT may include an error due to the PEP. In contrast, as described above, in the case where the first electronic device 100 calculates a blood pressure value based on a time difference between a first PPG signal and a second PPG signal, there may be prevented an error due to the PEP when a blood pressure value is calculated.

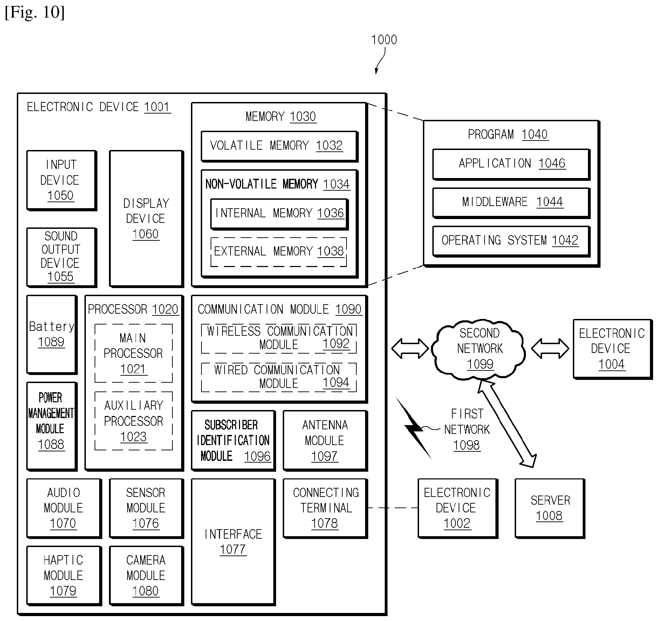

FIG. 10 is a block diagram illustrating an electronic device in a network environment according to various embodiments of the disclosure.

Referring to FIG. 10, the electronic device 1001 in the network environment 1000 may communicate with an electronic device 1002 via a first network 1098 (e.g., a short-range wireless communication network), or an electronic device 1004 or a server 1008 via a second network 1099 (e.g., a long-range wireless communication network). According to an embodiment, the electronic device 1001 may communicate with the electronic device 1004 via the server 1008. According to an embodiment, the electronic device 1001 may include a processor 1020, memory 1030, an input device 1050, a sound output device 1055, a display device 1060, an audio module 1070, a sensor module 1076, an interface 1077, a haptic module 1079, a camera module 1080, a power management module 1088, a battery 1089, a communication module 1090, a subscriber identification module (SIM) 1096, or an antenna module 1097. In some embodiments, at least one (e.g., the display device 1060 or the camera module 1080) of the components may be omitted from the electronic device 1001, or one or more other components may be added in the electronic device 1001. In some embodiments, some of the components may be implemented as single integrated circuitry. For example, the sensor module 1076 (e.g., a fingerprint sensor, an iris sensor, or an illuminance sensor) may be implemented as embedded in the display device 1060 (e.g., a display).

The processor 1020 may execute, for example, software (e.g., a program 1040) to control at least one other component (e.g., a hardware or software component) of the electronic device 1001 coupled with the processor 1020, and may perform various data processing or computation. According to one embodiment, as at least part of the data processing or computation, the processor 1020 may load a command or data received from another component (e.g., the sensor module 1076 or the communication module 1090) in volatile memory 1032, process the command or the data stored in the volatile memory 1032, and store resulting data in non-volatile memory 1034. According to an embodiment, the processor 1020 may include a main processor 1021 (e.g., a central processing unit (CPU) or an application processor (AP)), and an auxiliary processor 1023 (e.g., a graphics processing unit (GPU), an image signal processor (ISP), a sensor hub processor, or a communication processor (CP)) that is operable independently from, or in conjunction with, the main processor 1021. Additionally or alternatively, the auxiliary processor 1023 may be adapted to consume less power than the main processor 1021, or to be specific to a specified function. The auxiliary processor 1023 may be implemented as separate from, or as part of the main processor 1021.

The auxiliary processor 1023 may control at least some of functions or states related to at least one component (e.g., the display device 1060, the sensor module 1076, or the communication module 1090) among the components of the electronic device 1001, instead of the main processor 1021 while the main processor 1021 is in an inactive (e.g., sleep) state, or together with the main processor 1021 while the main processor 1021 is in an active state (e.g., executing an application). According to an embodiment, the auxiliary processor 1023 (e.g., an image signal processor or a communication processor) may be implemented as part of another component (e.g., the camera module 1080 or the communication module 1090) functionally related to the auxiliary processor 1023.

The memory 1030 may store various data used by at least one component (e.g., the processor 1020 or the sensor module 1076) of the electronic device 1001. The various data may include, for example, software (e.g., the program 1040) and input data or output data for a command related thererto. The memory 1030 may include the volatile memory 1032 or the non-volatile memory 1034.

The program 1040 may be stored in the memory 1030 as software, and may include, for example, an operating system (OS) 1042, middleware 1044, or an application 1046.

The input device 1050 may receive a command or data to be used by other component (e.g., the processor 1020) of the electronic device 1001, from the outside (e.g., a user) of the electronic device 1001. The input device 1050 may include, for example, a microphone, a mouse, a keyboard, or a digital pen (e.g., a stylus pen).

The sound output device 1055 may output sound signals to the outside of the electronic device 1001. The sound output device 1055 may include, for example, a speaker or a receiver. The speaker may be used for general purposes, such as playing multimedia or playing record, and the receiver may be used for an incoming calls. According to an embodiment, the receiver may be implemented as separate from, or as part of the speaker.

The display device 1060 may visually provide information to the outside (e.g., a user) of the electronic device 1001. The display device 1060 may include, for example, a display, a hologram device, or a projector and control circuitry to control a corresponding one of the display, hologram device, and projector. According to an embodiment, the display device 1060 may include touch circuitry adapted to detect a touch, or sensor circuitry (e.g., a pressure sensor) adapted to measure the intensity of force incurred by the touch.

The audio module 1070 may convert a sound into an electrical signal and vice versa. According to an embodiment, the audio module 1070 may obtain the sound via the input device 1050, or output the sound via the sound output device 1055 or a headphone of an external electronic device (e.g., an electronic device 1002) directly (e.g., wiredly) or wirelessly coupled with the electronic device 1001.

The sensor module 1076 may detect an operational state (e.g., power or temperature) of the electronic device 1001 or an environmental state (e.g., a state of a user) external to the electronic device 1001, and then generate an electrical signal or data value corresponding to the detected state. According to an embodiment, the sensor module 1076 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 1077 may support one or more specified protocols to be used for the electronic device 1001 to be coupled with the external electronic device (e.g., the electronic device 1002) directly (e.g., wiredly) or wirelessly. According to an embodiment, the interface 1077 may include, for example, a high definition multimedia interface (HDMI), a universal serial bus (USB) interface, a secure digital (SD) card interface, or an audio interface.

A connecting terminal 1078 may include a connector via which the electronic device 1001 may be physically connected with the external electronic device (e.g., the electronic device 1002). According to an embodiment, the connecting terminal 1078 may include, for example, a HDMI connector, a USB connector, a SD card connector, or an audio connector (e.g., a headphone connector).

The haptic module 1079 may convert an electrical signal into a mechanical stimulus (e.g., a vibration or a movement) or electrical stimulus which may be recognized by a user via his tactile sensation or kinesthetic sensation. According to an embodiment, the haptic module 1079 may include, for example, a motor, a piezoelectric element, or an electric stimulator.

The camera module 1080 may capture an image or moving images. According to an embodiment, the camera module 1080 may include one or more lenses, image sensors, image signal processors, or flashes.

The power management module 1088 may manage power supplied to the electronic device 1001. According to one embodiment, the power management module 1088 may be implemented as at least part of, for example, a power management integrated circuit (PMIC).

The battery 1089 may supply power to at least one component of the electronic device 1001. According to an embodiment, the battery 1089 may include, for example, a primary cell which is not rechargeable, a secondary cell which is rechargeable, or a fuel cell.

The communication module 1090 may support establishing a direct (e.g., wired) communication channel or a wireless communication channel between the electronic device 1001 and the external electronic device (e.g., the electronic device 1002, the electronic device 1004, or the server 1008) and performing communication via the established communication channel. The communication module 1090 may include one or more communication processors that are operable independently from the processor 1020 (e.g., the application processor (AP)) and supports a direct (e.g., wired) communication or a wireless communication. According to an embodiment, the communication module 1090 may include a wireless communication module 1092 (e.g., a cellular communication module, a short-range wireless communication module, or a global navigation satellite system (GNSS) communication module) or a wired communication module 1094 (e.g., a local area network (LAN) communication module or a power line communication (PLC) module). A corresponding one of these communication modules may communicate with the external electronic device via the first network 1098 (e.g., a short-range communication network, such as Bluetooth^{™}, wireless-fidelity (Wi-Fi) direct, or infrared data association (IrDA)) or the second network 1099 (e.g., a long-range communication network, such as a cellular network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 1092 may identify and authenticate the electronic device 1001 in a communication network, such as the first network 1098 or the second network 1099, using subscriber information (e.g., international mobile subscriber identity (IMSI)) stored in the subscriber identification module 1096.

The antenna module 1097 may transmit or receive a signal or power to or from the outside (e.g., the external electronic device) of the electronic device 1001. According to an embodiment, the antenna module 1097 may include an antenna including a radiating element composed of a conductive material or a conductive pattern formed in or on a substrate (e.g., PCB). According to an embodiment, the antenna module 1097 may include a plurality of antennas. In such a case, at least one antenna appropriate for a communication scheme used in the communication network, such as the first network 1098 or the second network 1099, may be selected, for example, by the communication module 1090 (e.g., the wireless communication module 1092) from the plurality of antennas. The signal or the power may then be transmitted or received between the communication module 1090 and the external electronic device via the selected at least one antenna. According to an embodiment, another component (e.g., a radio frequency integrated circuit (RFIC)) other than the radiating element may be additionally formed as part of the antenna module 1097.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 1001 and the external electronic device 1004 via the server 1008 coupled with the second network 1099. Each of the electronic devices 1002 and 1004 may be a device of a same type as, or a different type, from the electronic device 1001. According to an embodiment, all or some of operations to be executed at the electronic device 1001 may be executed at one or more of the external electronic devices 1002, 1004, or 1008. For example, if the electronic device 1001 should perform a function or a service automatically, or in response to a request from a user or another device, the electronic device 1001, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 1001. The electronic device 1001 may provide the outcome, with or without further processing of the outcome, as at least part of a reply to the request. To that end, a cloud computing, distributed computing, or client-server computing technology may be used, for example.

The electronic device according to various embodiments may be one of various types of electronic devices. The electronic devices may include, for example, a portable communication device (e.g., a smartphone), a computer device, a portable multimedia device, a portable medical device, a camera, a wearable device, or a home appliance. According to an embodiment of the disclosure, the electronic devices are not limited to those described above.

It should be appreciated that various embodiments of the disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things, unless the relevant context clearly indicates otherwise. As used herein, each of such phrases as "A or B," "at least one of A and B," "at least one of A or B," "A, B, or C," "at least one of A, B, and C," and "at least one of A, B, or C," may include any one of, or all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," "coupled to," "connected with," or "connected to" another element (e.g., a second element), it means that the element may be coupled with the other element directly (e.g., wiredly), wirelessly, or via a third element.

As used herein, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., the program 1040) including one or more instructions that are stored in a storage medium (e.g., internal memory 1036 or external memory 1038) that is readable by a machine (e.g., the electronic device 1001). For example, a processor(e.g., the processor 1020) of the machine (e.g., the electronic device 1001) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a compiler or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment, a method according to various embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., PlayStore^{™}), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each component (e.g., a module or a program) of the above-described components may include a single entity or multiple entities. According to various embodiments, one or more of the above-described components may be omitted, or one or more other components may be added. Alternatively or additionally, a plurality of components (e.g., modules or programs) may be integrated into a single component. In such a case, according to various embodiments, the integrated component may perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

According to embodiments of the disclosure, a blood pressure value may be calculated based on a PWV algorithm by using only a PPG sensor without an ECG sensor. Besides, a variety of effects directly or indirectly understood through this disclosure may be provided.

While the disclosure has been shown and described with reference to various embodiments thereof, it will be understood by those skilled in the art that various changes in form and details may be made therein without departing from the scope of the disclosure as defined by the appended claims.

## Claims

1. An electronic device (100) comprising:
a housing (100H):
a first photoplethysmography, PPG, sensor (110) exposed to a first portion of the housing (100H) and facing a first body portion of a user to measure a pulse wave;
an input circuit (130) configured to receive an input associated with a request for providing the first blood pressure value;
a sensor circuit (150) configured to sense a movement of the electronic device (100);
a wireless communication circuit (120) positioned within the housing (100H);a processor (170) positioned within the housing (100H) and operatively connected with the first PPG sensor (110), the input circuit, the sensor circuit, and the wireless communication circuit (120); and
a memory (160) positioned within the housing (100H) and operatively connected with the processor (170),
wherein the memory (160) stores instructions that, when executed, cause the processor (170) to:
monitor a first pulse signal measured by the first PPG sensor (110),
receive a second pulse signal measured by an external electronic device by using the wireless communication circuit (120),
obtain a time difference between the first pulse signal and the second pulse signal, based at least on the monitored first pulse signal and the received second pulse signal, and
provide a first blood pressure value by using a pulse wave velocity, PWV, algorithm, based at least on the obtained time difference,
**characterised in that**
the instructions further cause the processor (170) to:
obtain movement information of the electronic device (170) by using the sensor circuit (150),
determine whether the movement of the electronic device (100) is a specified strength or greater, based at least partially on the movement information, and
output information indicating that it is impossible to provide the first blood pressure value, through the user interface (140), when it is determined that the movement of the electronic device (100) is the specified strength or greater and the input is received within a specified time.

2. The electronic device (100) of claim 1, wherein the second pulse signal comprises a signal which the external electronic device measures from a second body portion, the second body portion spaced from the first body portion as much as a given distance, by using a second PPG sensor included in the external electronic device.

3. The electronic device (100) of claim 1, further comprising:
a user interface (140) disposed at a second portion of the housing (100H),
wherein the instructions further cause the processor (170) to:
display guide information associated with the provided first blood pressure value on the user interface.

4. The electronic device (100) of claim 1, wherein the instructions further cause the processor (170) to:
emit a light to the first body portion by using the first PPG sensor (110); and
adjust a sensitivity of the first PPG sensor (110) based on a reflection light of the emitted light.

5. A method of obtaining a blood pressure by an electronic device, the method comprising:
obtaining (510) a first pulse signal corresponding to a pulse wave measured at a first body portion of a user by using a first photoplethysmography, PPG, sensor facing the first body portion;
receiving (520), from an external electronic device including a second PPG sensor, a second pulse signal corresponding to a pulse wave, which the external electronic device measures at a second body portion of the user spaced from the first body portion as much as a given distance by using the second PPG sensor;
obtaining (530) a time difference between the obtained first pulse signal and the received second pulse signal;
obtaining (540) a first blood pressure value, based at least on the obtained time difference; and
providing (550) the first blood pressure value through an output device,
**characterised in that** the method further comprises:
obtaining movement information of the electronic device;
determining whether a movement of the electronic device is a specified strength or greater, based at least partially on the obtained movement information;
receiving an input associated with a blood pressure obtaining request; and
outputting information indicating that it is impossible to obtain a blood pressure, through the output device, when the input is received within a specified time after it is determined that the movement of the electronic device is the specified strength or greater.

6. The method of claim 5, wherein the obtaining of the first blood pressure value includes:
obtaining (510) the first blood pressure value corresponding to the obtained time difference, based at least partially on relationship information between a blood pressure value and the obtained time difference.

7. The method of claim 5, further comprising:
estimating a second blood pressure value by using a pulse wave analysis (PWA) algorithm, based on the first pulse signal;
obtaining a third blood pressure value obtained by using the PWA algorithm based on the second pulse signal from the external electronic device; and
verifying at least one of an accuracy of the first blood pressure value or an abnormal cardiovascular status of the user based at least partially on the second blood pressure value or the third blood pressure value.

8. The method of claim 5, wherein the receiving (520) of the second pulse signal includes:
communicating with the external electronic device to receive the second pulse signal synchronized with the first pulse signal.

9. The method of claim 5,
wherein the external electronic device further comprises an electrocardiography, ECG sensor, and
wherein the method further comprises receiving, from the external electronic device, a second blood pressure value calculated based on an ECG signal from the ECG sensor and the second pulse signal.

10. The method of claim 9, wherein the ECG signal indicates a time point when an electrical stimulation of ventricles of a heart starts.

11. The method of claim 5, wherein the obtaining (530) of the time difference between the obtained first pulse signal and the received second pulse signal comprises:
detecting a peak of the obtained first pulse signal,
detecting a peak of the received second pulse signal, and
calculating a time difference between the detected peaks.

## Patentansprüche

1. Elektronische Vorrichtung (100), die Folgendes umfasst:
ein Gehäuse (100H):
einen ersten Photoplethysmographie-, PPG,-Sensor (110), der zu einem ersten Abschnitt des Gehäuses (100H) freigelegt und einem ersten Körperabschnitt eines Benutzers zugewandt ist, um eine Pulswelle zu messen;
eine Eingabeschaltung (130), die konfiguriert ist, um eine Eingabe zu empfangen, die mit einer Anforderung zur Bereitstellung des ersten Blutdruckwerts verbunden ist;
eine Sensorschaltung (150), die konfiguriert ist, um eine Bewegung der elektronischen Vorrichtung (100) zu erfassen;
eine drahtlose Kommunikationsschaltung (120), die innerhalb des Gehäuses (100H) positioniert ist;
einen Prozessor (170), der innerhalb des Gehäuses (100H) positioniert ist und operativ mit dem ersten PPG-Sensor (110), der Eingabeschaltung, der Sensorschaltung und der drahtlosen Kommunikationsschaltung (120) verbunden ist; und
einen Speicher (160), der innerhalb des Gehäuses (100H) positioniert und operativ mit dem Prozessor (170) verbunden ist,
wobei der Speicher (160) Instruktionen speichert, die, wenn sie ausgeführt werden, den Prozessor (170) dazu veranlassen:
ein erstes Pulssignal zu überwachen, das von dem ersten PPG-Sensor (110) gemessen wird,
ein zweites Pulssignal zu empfangen, das von einer externen elektronischen Vorrichtung unter Verwendung der drahtlosen Kommunikationsschaltung (120) gemessen wird,
eine Zeitdifferenz zwischen dem ersten Pulssignal und dem zweiten Pulssignal basierend zumindest auf dem überwachten ersten Pulssignal und dem empfangenen zweiten Pulssignal zu erhalten, und
einen ersten Blutdruckwert unter Verwendung eines Pulswellengeschwindigkeits-, PWV,-Algorithmus zumindest auf der Grundlage der erhaltenen Zeitdifferenz bereitzustellen,
**dadurch gekennzeichnet, dass** die Instruktionen ferner den Prozessor (170) veranlassen zum:
Erhalten von Bewegungsinformationen der elektronischen Vorrichtung (170) unter Verwendung der Sensorschaltung (150),
Bestimmen, ob die Bewegung der elektronischen Vorrichtung (100) eine bestimmte Stärke oder mehr ist, zumindest teilweise basierend auf den Bewegungsinformationen, und
Ausgeben von Informationen über die Benutzerschnittstelle (140), die anzeigen, dass es unmöglich ist, den ersten Blutdruckwert bereitzustellen, wenn bestimmt wird, dass die Bewegung der elektronischen Vorrichtung (100) die angegebene Stärke oder mehr ist und die Eingabe innerhalb einer bestimmten Zeit empfangen wird.

2. Elektronische Vorrichtung (100) nach Anspruch 1, wobei das zweite Pulssignal ein Signal umfasst, das die externe elektronische Vorrichtung von einem zweiten Körperabschnitt misst, wobei der zweite Körperabschnitt vom ersten Körperabschnitt in einem bestimmten Abstand beabstandet ist, indem ein zweiter PPG-Sensor verwendet wird, der in der externen elektronischen Vorrichtung enthalten ist.

3. Elektronische Vorrichtung (100) nach Anspruch 1, die ferner umfasst:
eine Benutzerschnittstelle (140), die an einem zweiten Abschnitt des Gehäuses (100H) angeordnet ist,
wobei die Instruktionen den Prozessor (170) ferner veranlassen zum:
Anzeigen von Führungsinformationen, die mit dem bereitgestellten ersten Blutdruckwert auf der Benutzerschnittstelle verbunden sind.

4. Elektronische Vorrichtung (100) nach Anspruch 1, wobei die Instruktionen den Prozessor (170) ferner veranlassen zum:
Emittieren eines Lichts an den ersten Körperabschnitt unter Verwendung des ersten PPG-Sensors (110); und
Einstellen einer Empfindlichkeit des ersten PPG-Sensors (110) basierend auf einem Reflexionslicht des emittierten Lichts.

5. Verfahren zum Erhalten des Blutdrucks durch eine elektronische Vorrichtung, wobei das Verfahren umfasst:
Erhalten (510) eines ersten Pulssignals, das einer Pulswelle entspricht, die an einem ersten Körperabschnitt eines Benutzers unter Verwendung eines ersten Photoplethysmographie-, PPG-,Sensors gemessen wird, der dem ersten Körperabschnitt zugewandt ist;
Empfangen (520), von einer externen elektronischen Vorrichtung, die einen zweiten PPG-Sensor enthält, eines zweiten Pulssignals, das einer Pulswelle entspricht, die die externe elektronische Vorrichtung an einem zweiten Körperabschnitt des Benutzers misst, der von dem ersten Körperabschnitt in einem bestimmten Abstand beabstandet ist, indem der zweite PPG-Sensor verwendet wird;
Erhalten (530) einer Zeitdifferenz zwischen dem erhaltenen ersten Pulssignal und dem empfangenen zweiten Pulssignal;
Erhalten (540) eines ersten Blutdruckwertes basierend zumindest auf der erhaltenen Zeitdifferenz; und
Bereitstellen (550) des ersten Blutdruckwerts über eine Ausgabevorrichtung,
**dadurch gekennzeichnet, dass** das Verfahren ferner umfasst:
Erhalten von Bewegungsinformationen der elektronischen Vorrichtung;
Bestimmen, ob eine Bewegung der elektronischen Vorrichtung eine bestimmte Stärke oder mehr ist, zumindest teilweise basierend auf der erhaltenen Bewegungsinformation;
Empfangen einer Eingabe, die mit einer Blutdruckerhaltungsanforderung verbunden ist; und
Ausgeben von Informationen über die Ausgabevorrichtung, die anzeigen, dass es unmöglich ist, einen Blutdruck zu erhalten, wenn die Eingabe innerhalb einer bestimmten Zeit empfangen wird, nachdem bestimmt wurde, dass die Bewegung der elektronischen Vorrichtung die bestimmte Stärke oder mehr ist.

6. Verfahren nach Anspruch 5, wobei das Erhalten des ersten Blutdruckwertes umfasst:
Erhalten (510) des ersten Blutdruckwertes, der der erhaltenen Zeitdifferenz entspricht, zumindest teilweise basierend auf Beziehungsinformationen zwischen einem Blutdruckwert und der erhaltenen Zeitdifferenz.

7. Verfahren nach Anspruch 5, das ferner umfasst:
Schätzen eines zweiten Blutdruckwertes unter Verwendung eines Pulswellenanalyse-(PWA)-Algorithmus basierend auf dem ersten Pulssignal;
Erhalten eines dritten Blutdruckwertes, der durch Verwendung des PWA-Algorithmus basierend auf dem zweiten Pulssignal von der externen elektronischen Vorrichtung erhalten wird; und
Überprüfen mindestens eines einer Genauigkeit des ersten Blutdruckwertes oder eines abnormalen kardiovaskulären Status des Benutzers zumindest teilweise basierend auf dem zweiten Blutdruckwert oder dem dritten Blutdruckwert.

8. Verfahren nach Anspruch 5, wobei das Empfangen (520) des zweiten Pulssignals Folgendes umfasst:
Kommunizieren mit der externen elektronischen Vorrichtung, um das zweite Pulssignal synchronisiert mit dem ersten Pulssignal zu empfangen.

9. Verfahren nach Anspruch 5,
wobei die externe elektronische Vorrichtung ferner einen Elektrokardiographie-, ECG-,Sensor umfasst, und
wobei das Verfahren ferner das Empfangen, von der externen elektronischen Vorrichtung, eines zweiten Blutdruckwertes umfasst, der basierend auf einem ECG-Signal von dem ECG-Sensor und dem zweiten Pulssignal berechnet wird.

10. Verfahren nach Anspruch 9, wobei das ECG-Signal einen Zeitpunkt angibt, an dem eine elektrische Stimulation der Herzkammern beginnt.

11. Verfahren nach Anspruch 5, wobei das Erhalten (530) der Zeitdifferenz zwischen dem erhaltenen ersten Pulssignal und dem empfangenen zweiten Pulssignal Folgendes umfasst:
Erfassen einer Spitze des erhaltenen ersten Pulssignals,
Erfassen einer Spitze des empfangenen zweiten Pulssignals und
Berechnen einer Zeitdifferenz zwischen den erkannten Spitzen.

## Revendications

1. Dispositif électronique (100) comprenant :
un boîtier (100H) :
un premier capteur de photopléthysmographie, PPG, (110) exposé à une première partie du boîtier (100H) et faisant face à une première partie de corps d'un utilisateur pour mesurer une onde d'impulsion ;
un circuit d'entrée (130) configuré pour recevoir une entrée associée à une demande de fourniture de la première valeur de pression artérielle ;
un circuit de capteur (150) configuré pour détecter un mouvement du dispositif électronique (100) ;
un circuit de communication sans fil (120) placé dans le boîtier (100H) ;
un processeur (170) placé dans le boîtier (100H) et connecté de manière opérationnelle au premier capteur PPG (110), au circuit d'entrée, au circuit de capteur et au circuit de communication sans fil (120) ; et
une mémoire (160) positionnée dans le boîtier (100H) et connectée de manière opérationnelle au processeur (170),
dans lequel la mémoire (160) stocke des instructions qui, lorsqu'elles sont exécutées, amènent le processeur (170) à :
surveiller un premier signal d'impulsion mesuré par le premier capteur PPG (110),
recevoir un deuxième signal d'impulsion mesuré par un dispositif électronique externe en utilisant le circuit de communication sans fil (120),
obtenir une différence de temps entre le premier signal d'impulsion et le deuxième signal d'impulsion, sur la base au moins du premier signal d'impulsion surveillé et du deuxième signal d'impulsion reçu, et
fournir une première valeur de pression artérielle en utilisant un algorithme de vitesse d'onde d'impulsion, PWV, basé au moins sur la différence de temps obtenue, **caractérisé en ce que** les instructions amènent en outre le processeur (170) à :
obtenir des informations de mouvement du dispositif électronique (170) en utilisant le circuit de capteur (150),
déterminer si le mouvement du dispositif électronique (100) est d'une force spécifiée ou supérieure, en se basant au moins partiellement sur les informations de mouvement, et
émettre des informations indiquant qu'il est impossible de fournir la première valeur de pression artérielle, par l'intermédiaire de l'interface utilisateur (140), lorsqu'il est déterminé que le mouvement du dispositif électronique (100) est de la force spécifiée ou supérieure et que l'entrée est reçue dans un temps spécifié.

2. Dispositif électronique (100) de la revendication 1, dans lequel le deuxième signal d'impulsion comprend un signal que le dispositif électronique externe mesure à partir d'une deuxième partie de corps, la deuxième partie de corps étant espacée de la première partie de corps jusqu'à une distance donnée, en utilisant un deuxième capteur PPG inclus dans le dispositif électronique externe.

3. Dispositif électronique (100) de la revendication 1, comprenant en outre :
une interface utilisateur (140) disposée sur une deuxième partie du boîtier (100H),
dans lequel les instructions amènent en outre le processeur (170) à :
afficher sur l'interface utilisateur des informations de guidage associées à la première valeur de pression artérielle fournie.

4. Dispositif électronique (100) de la revendication 1, dans lequel les instructions amènent en outre le processeur (170) à :
émettre une lumière sur la première partie de corps à l'aide du premier capteur PPG (110) ; et
ajuster une sensibilité du premier capteur PPG (110) en fonction de la lumière de réflexion de la lumière émise.

5. Procédé d'obtention d'une pression artérielle par un dispositif électronique, le procédé comprenant :
obtenir (510) un premier signal d'impulsion correspondant à une onde d'impulsion mesurée sur une première partie de corps d'un utilisateur à l'aide d'un premier capteur de photopléthysmographie, PPG, faisant face à la première partie du corps ;
recevoir (520), depuis un dispositif électronique externe comprenant un deuxième capteur PPG, un deuxième signal d'impulsion correspondant à une onde d'impulsion que le dispositif électronique externe mesure au niveau d'une deuxième partie de corps de l'utilisateur espacée de la première partie de corps jusqu'à une distance donnée à l'aide du deuxième capteur PPG ;
obtenir (530) une différence de temps entre le premier signal d'impulsion obtenu et le deuxième signal d'impulsion reçu ;
obtenir (540) une première valeur de pression artérielle, sur la base au moins de la différence de temps obtenue ; et
fournir (550) la première valeur de pression artérielle par l'intermédiaire d'un dispositif de sortie,
**caractérisé en ce que** le procédé comprend en outre :
obtenir des informations de mouvement du dispositif électronique ;
déterminer si un mouvement du dispositif électronique est d'une force spécifiée ou supérieure, en se basant au moins partiellement sur les informations de mouvement obtenues ;
recevoir une entrée associée à une demande d'obtention de la pression artérielle ; et
émettre des informations indiquant qu'il est impossible d'obtenir une pression artérielle, par l'intermédiaire du dispositif de sortie, lorsque l'entrée est reçue dans un temps spécifié après qu'il a été déterminé que le mouvement du dispositif électronique est de la force spécifiée ou supérieure.

6. Procédé de la revendication 5, dans lequel l'obtention de la première valeur de pression artérielle comprend :
obtenir (510) la première valeur de pression artérielle correspondant à la différence de temps obtenue, sur la base au moins partiellement d'informations de relation entre une valeur de pression artérielle et la différence de temps obtenue.

7. Procédé de la revendication 5, comprenant en outre :
estimer une deuxième valeur de pression artérielle à l'aide d'un algorithme d'analyse d'onde d'impulsion (PWA), sur la base du premier signal d'impulsion ;
obtenir une troisième valeur de pression artérielle obtenue à l'aide de l'algorithme PWA sur la base du deuxième signal d'impulsion provenant du dispositif électronique externe ; et
vérifier au moins l'un de l'exactitude de la première valeur de pression artérielle ou d'un état cardiovasculaire anormal de l'utilisateur, en se basant au moins partiellement sur la deuxième valeur de pression artérielle ou la troisième valeur de pression artérielle.

8. Procédé de la revendication 5, dans lequel la réception (520) du deuxième signal d'impulsion comprend :
communiquer avec le dispositif électronique externe pour recevoir le deuxième signal d'impulsion synchronisé avec le premier signal d'impulsion.

9. Procédé de la revendication 5,
dans lequel le dispositif électronique externe comprend en outre un capteur d'électrocardiographie, ECG, et
dans lequel le procédé comprend en outre recevoir, du dispositif électronique externe, une deuxième valeur de pression artérielle calculée sur la base d'un signal ECG provenant du capteur ECG et du deuxième signal d'impulsion.

10. Procédé de la revendication 9, dans lequel le signal ECG indique un moment où une stimulation électrique des ventricules d'un coeur commence.

11. Procédé de la revendication 5, dans lequel l'obtention (530) de la différence de temps entre le premier signal d'impulsion obtenu et le deuxième signal d'impulsion reçu comprend :
détecter une crête du premier signal d'impulsion obtenu,
détecter une crête du deuxième signal d'impulsion reçu, et
calculer une différence de temps entre les crêtes détectées.
